# EUROPEAN PATENT SPECIFICATION

(11) **EP 2 964 395 B1**
(45) Date of publication and mention of the grant of the patent: **08.05.2019**
(21) Application number: 14712481.2
(22) Date of filing: 05.03.2014
(51) Int. Cl.: B05C 17/005, B05C 17/01, G01F 11/02, A61M 3/00, A61M 5/19, A61M 5/315, A61B 17/00, A61M 5/24, A61M 5/50, A61M 5/31

(54) **DOUBLE-CARTRIDGE SYRINGE FOR MIXING AND DISPENSING ADIPOSE TISSUE WITH ADDITIVE**
DOPPELKARTUSCHENSPRITZE ZUM MISCHEN UND AUSTRAGEN VON FETTGEWEBE MIT ZUSÄTZEN
SERINGUE À DOUBLE CARTOUCHE POUR MÉLANGER ET INJECTER TISSU ADIPEUX AVEC ADDITIF

(30) Priority: 07.03.2013 US 201361774085 P
(43) Date of publication of application: 13.01.2016
(73) Proprietor: ALLERGAN, INC., Irvine, CA 92612 (US)
(72) Inventor: SCHWAB, Justin, J., Santa Barbara, California 93105 (US); KAYDA, Edwin, J., Santa Barbara, California 93109 (US); BERTOLOTE, Tiago, CH-1202 Geneve (CH)
(74) Representative: Hoffmann Eitle
(86) International application number: PCT/US2014/020738
(87) International publication number: WO 2014/138226

(56) References cited:
- US-A- 5 137 181
- US-A- 5 520 658
- US-A- 5 722 829
- US-A- 6 047 861
- US-A1- 2003 233 067
- US-A1- 2007 191 781
- US-A1- 2009 131 886

## Description

The present invention generally relates to fat grafting procedures and more specifically relates to devices and methods for combining and dispensing adipose tissue and biocompatible additives for use in fat grafting procedures.

Autologous fat transfer (AFT), also known as fat grafting, is a process by which fat is harvested from one part of a human body and injected into another part of the same person's body where additional bulk may be needed or desired for cosmetic and/or aesthetic purposes. Clinical applications for autologous fat transfer are expanding rapidly with recent reported use in breast reconstruction and augmentation, buttock enhancement, treatment of congenital tissue defects, facial reconstruction, and skin rejuvenation. Although this is a very attractive approach and there is an increased trend in replacement of soft tissue volume with AFT, typical survival rates of grafted fat may be poor and overall results may not be satisfactory.

U.S. Patent Application Publication No. 20110202014 discloses a fat graft syringe assembly for delivering small amounts of fat graft material to treat delicate anatomical areas.

WO 2008148071 discloses kits, tools, and methods are described for harvesting, processing, and using injectable dermis in volume filling procedures.

WO 200903135 discloses system for harvesting fat through liposuction, concentrating the aspirate so obtained, and then re-injecting the concentrated fat into a A device as described in the preamble of claim 1 is know from document US 5 520 658 A.

There still remains a need for improved devices and methods for use in fat grafting procedures.

### SUMMARY

Accordingly, a device for introducing into a target region of a patient, a combination of adipose tissue and an additive, is provided. The device generally comprises a fat cartridge for containing an amount of processed or unprocessed adipose tissue or adipose derived material, and an additive cartridge for containing an additive to be combined with the adipose material. The device further comprises a housing configured to receive, for example, in a side-by-side manner, the fat cartridge and additive cartridge, and a mixing tip on the housing, the mixing tip including a distal end for receiving a cannula or needle. The device further comprises a plunger assembly including a fat cartridge plunger and an additive cartridge plunger, configured to be slidably received in the fat cartridge and the additive cartridge, respectively, and for applying force to the contents of the respective cartridges for moving the contents into the mixing tip and eventually out of the cannula or needle.

In some embodiments, the fat cartridge plunger and the additive cartridge plunger are fixed with respect to each other, for example, at a proximal end of the plunger assembly.

In some embodiments, the housing is configured to receive the fat cartridge and the additive cartridge in a longitudinally slidable manner. In other embodiments, the housing is configured to receive the fat cartridge and the additive cartridge in a lateral manner.

In one aspect of the invention, the device further comprises a mechanism for preventing extrusion of additive from the additive cartridge without coextrusion of fat from the fat cartridge.

In another aspect of the invention, a device is provided for administering a formulation comprising an adipose tissue and an additive wherein the device generally comprises (a) a first cartridge for containing an adipose tissue, (b) a second cartridge for containing an additive to be added to and mixed with the adipose tissue and (c) a housing configured to substantially enclose in a side-by-side manner the first and the second cartridges. In addition, the device comprises (d) a mixing tip on the housing, wherein the mixing tip includes (i) a chamber or lumen, (ii) a proximal end for receiving and conducting into the lumen the adipose tissue and the additive for mixing within the lumen, and (iii) a distal end for receiving a cannula or needle through which the formulation can be administered. Further still, the device may comprise (e) a plunger assembly including a first cartridge plunger and a second cartridge plunger, the plunger assembly configured to be slidably received in the first cartridge and in the second cartridge and the plunger assembly when actuated acting to push the adipose tissue, by the action of the first cartridge plunger, from the first cartridge into the lumen and to push the additive, by the action of the second cartridge plunger, from the second cartridge into the lumen, for the mixing therein.

In another embodiment, the device comprises a ratchet mechanism, including a lever and at least one toothed element connected to the plunger assembly, the ratchet mechanism configured to force the first cartridge plunger and the second cartridge plunger in a distal direction upon manual pressure applied to the lever.

In some embodiments, a device is provided with a ratchet mechanism to provide more advanced precision of injection amount.

Each and every feature described herein, and each and every combination of two or more of such features, is included within the scope of the present invention provided that the features included in such a combination are not mutually inconsistent.

### BRIEF DESCRIPTION OF THE DRAWINGS

The present invention may be more clearly understood and the advantages thereof better appreciated by considering the below Detailed Description and accompanying Drawings of which:
Figure 1 is a perspective, exploded view of a device in accordance with one embodiment of the invention;
Figure 2 is a perspective view of the device of Figure 1, in a fully assembled condition.
Figure 3 is a perspective, exploded view of another device in accordance with another embodiment of the invention;
Figure 4 is a perspective view of the device of Figure 3, in a fully assembled condition.
Figure 5 is a perspective, exploded view of another device in accordance with yet another embodiment of the invention;
Figure 6 is a perspective view of the device of Figure 5, in a fully assembled condition;
Figures 7 and 8 are perspective views of yet another device in accordance with another embodiment of the invention, including an adjustable thumb piece loose for positioning (Figure 7) and engaged (Figure 8).
Figure 9 shows simplified views of a device of the invention including a variety of different mixing tips; and
Figure 10 is a simplified diagram of a feature of the invention for preventing misuse thereof.
Figure 11 is a perspective view of another embodiment of the invention including a ratchet mechanism.
Figures 12A-12D are depictions of sequential operation of the device shown in Figure 11.

### DETAILED DESCRIPTION

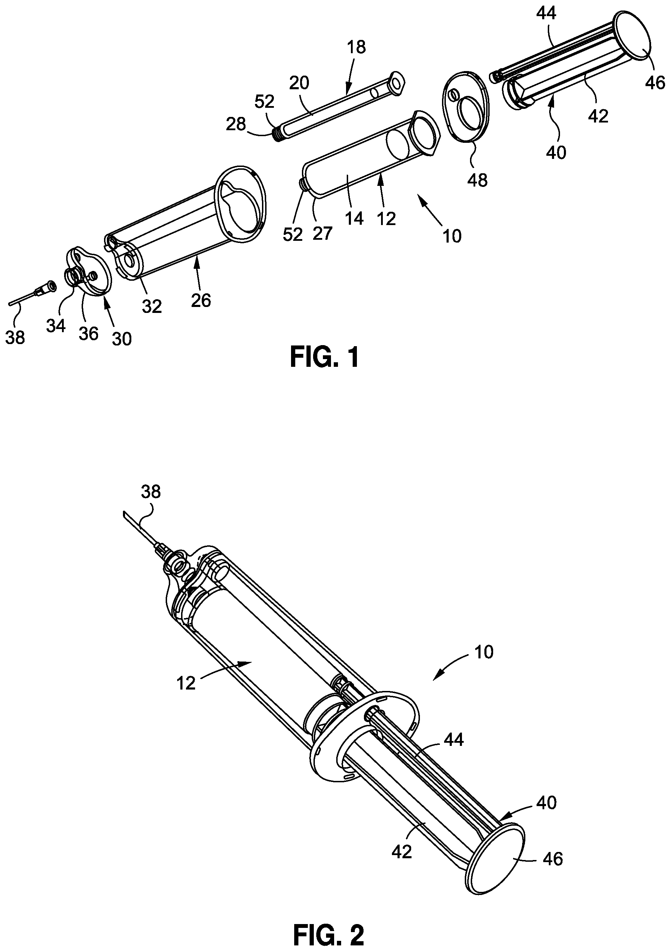

Referring now to Figures 1 and 2, in the shown embodiment, a dual cartridge syringe device, for injection of fat with an additive into the body is provided. The device 10 is generally structured to mix freshly harvested and/or processed fat with an additive for improved viability as it is injected into a patient, for example, for breast augmentation, body contouring, dermal filling, reconstructive purposes, or the like.

The device 10 generally comprises a first cartridge 12, hereinafter sometimes referred to as a fat cartridge 12, for containing an amount of processed or unprocessed adipose tissue 14 or adipose derived material, and a second cartridge 18, hereinafter sometimes referred to as an additive cartridge 18, for containing an additive 20 to be combined with the adipose tissue 14.

The adipose tissue may be in the form of freshly harvested or processed lipoaspirate, including adipocytes, adipose-derived stem cells, stromal vascular fraction cells, or combinations thereof.

Additives useful in within the scope of the present devices may be any material that may be mixed with cellular material, for example, living cells, for example, adipose tissue including adipose cells, and which is beneficial to maintaining the viability of the cellular material when mixed therewith and then injected or implanted in a body. Such additives may be in the form of hydrogels that enhance, promote or support cell proliferation or survival. Additives useful in the present devices are described, for example, in commonly owned U.S. Provisional Patent Application No. 61/586,589, filed on January 13, 2012, and entitled CROSSLINKED HYALURONIC ACID-COLLAGEN MATRICES FOR IMPROVING TISSUE GRAFT VIABILITY AND SOFT TISSUE AUGMENTATION, the entire disclosure of which is incorporated herein by this reference.

When injected or implanted *in vivo,* the hydrogel or a hydrogel composition may promote cell and/or tissue growth, including growth into the implant material. For example, a hydrogel or hydrogel composition may stimulate angiogenesis, neovascularization, adipogenesis, collagenesis, cell infiltration, tissue integration, and the like *in vivo.* Once injected or implanted into a soft tissue using the devices of the present invention, a combined hydrogel composition and fat material may stimulate angiogenesis, neovascularization, adipogenesis, and/or collagenesis. The hydrogel composition may comprise a hyaluronic acid component and a collagen component, for example, a hyaluronic acid component crosslinked to a collagen component.

Turning back now to Figures 1 and 2, the device 10 further comprises a housing 26 configured to receive, for example, in a side-by-side manner as shown, the fat cartridge 12 and additive cartridge 18. In some embodiments, the device may be structured such that the additive cartridge 18 is non-removable, or fixed with respect to the housing, as a means to prevent or discourage misuse of the additive.

In the shown embodiment, the fat cartridge 12 and additive cartridge 18 include luer end 27, 28, respectively. The housing 26 is configured to hold two distinct, for example, different, cartridge sizes, as shown. In one embodiment, the volume ratio of fat cartridge to additive cartridge is about 2:1, up to about 5:1. For example, in the shown embodiment, the fat cartridge 12 may be sized to contain between about 5 ml to about 60 ml, or more, of material, and the additive cartridge 18 may be sized to contain between about 1 ml to about 30 ml of material. Other ratios and sizes are contemplated and are considered to be within the scope of the present invention.

The device 10 may further include a tip 30 coupled to or integral with a distal end 32 of the housing 26. The tip 30 generally includes a lumen or chamber 36, a proximal end for receiving and conducting into the lumen 36 the adipose tissue 14 and the additive 20, which may be mixed or combined within the lumen 36. The tip 30 further includes a distal end structured for receiving a cannula or needle, for example, more specifically, a needle hub. Formulation comprising a mixture of adipose tissue and additive may be passed from the tip lumen 36 into the needle or cannula 40 and thereby administered to a patient. The tip 30 includes a distal end 34 for receiving a cannula or needle 40, for example, more specifically, a needle hub. The tip lumen or chamber 36 may be structured to cause mixing or combining of fat and additive as these materials are passed through the chamber 36 and into the cannula or needle 38. Suitable needle and cannula gauges useful in the present invention include, but are not limited to, for example, 14 gauge up to 32 gauge needle/cannulas. Needle/cannulas may be blunt end or sharp.

The device 10 further comprises a plunger assembly 40 including a fat cartridge plunger 42 and an additive cartridge plunger 44, configured to be slidably received in the fat cartridge 12 and the additive cartridge 18, respectively, and for applying force to the contents of the respective cartridges 12, 18 for moving the contents 14, 20 into the tip 30 and eventually out of the cannula or needle 38 and into a patient.

In this shown embodiment, the plunger assembly 40 is a unitary structure, for example, a unitary molded structure, with proximal portion 46 providing a coupling region between fat cartridge plunger 42 and additive cartridge plunger 44. Proximal portion 46 may be configured as a planar surface, a concave or convex surface, to accommodate a thumb of an operator of the device 10. As shown, the plunger assembly 40 may be configured to be slidably received in the first cartridge 12 and in the second cartridge 18 and the plunger assembly 40 when actuated acting to push the adipose tissue 14, by the action of the first cartridge plunger 42, from the first cartridge 12 into the tip lumen 36 and to push the additive 20, by the action of the second cartridge plunger 44, from the second cartridge 18 into the lumen 36, for the mixing therein.

For each fat grafting procedure, the fat cartridge 12 is filled with freshly harvested or processed adipose tissue and placed into or engaged with the housing 26. The additive cartridge 18 is filled with an additive is also placed into the housing 26. A housing top cover 48 is snapped over the two cartridges 12, 18 securing them firmly in place in the housing 26. The tip 30 is snapped onto the housing 26 using a suitable mechanism, for example, o-rings 52, to seal fat cartridge luer end 27 and additive cartridge luer end 28 to the tip 30.

In use, an operator loads the fat cartridge 12 containing freshly harvested and/or prepared fat, into the fat cartridge 12. The additive cartridge 18 may already be in place in the housing 26, or the operator engages the additive cartridge 18 with the housing 26 just prior to the procedure. The housing top cover 48 is snapped into the housing 26 which is engaged to the mixing tip 30. The plunger assembly 40 is inserted into the cartridges 12, 18. The operator places the needle or cannula tip into a target region of the patient and presses the proximal portion 46 of the plunger assembly 40 to cause extrusion of fat and additive into the target region.

In some embodiments, the tip 30 may be modular in that it can be provided in a variety of different configurations allowing for a variety of mixing configurations and requirements. In some embodiments, for example, the tip 30 is configured to include mixing elements such as helical static mixers, grooves, pins, or other structure within the chamber 36 to effect mixing of the fat and additive as these are being extruded through chamber 36.

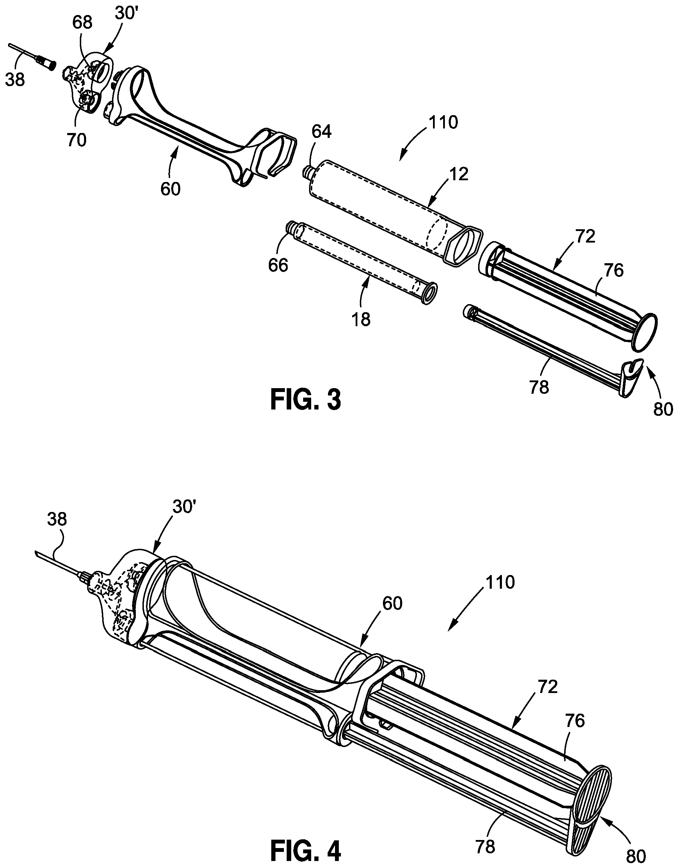

Turning now to Figures 3 and 4, another embodiment 110 of the present invention is shown.

Device 110 is similar to device 10 shown in Figures 1 and 2, with a difference being that instead of housing 26, device 110 includes open sided housing 60 which allows cartridges 12, 18 to be installed to mixing tip 30' by using luer thread or slip connector 64, 66 on cartridges 12, 18, rather than o-ring seals. Mixing tip 30' is similar to tip 30, with a major distinction being that mixing tip 30' includes coupling structure 68, 70 configured to engage luer connectors 64, 66, respectively, of cartridges 12, 18 rather than o-ring seals.

For example, for each surgical procedure, mixing tip 30' is snapped onto the open-sided housing 60. Additive cartridge 18 containing additive is inserted into the housing 60 through proximal end thereof, and is rotated until the luer feature 66 engage and tighten into the corresponding coupling structure 70 on tip 30'. Similarly, the fat cartridge 12 can be inserted into the housing 60 and snapped or rotated in place. The fat cartridge 12 seats into the tip 30' and uses the interference between luer end of the fat cartridge 12 and the corresponding connector 68 of tip 30' for creating a seal therebetween. Needle or cannula 38 may be installed to the tip 30 in a usual manner.

Also shown in Figures 3 and 4, is another feature different from the device 10 shown in Figures 1 and 2. Rather than plunger assembly 40, device 110 may include a plunger assembly 72 having separate, or separable, fat cartridge plunger 76 and additive cartridge plunger 78 which include coupling structure 80, such as a snap engagement or a slidable engagement, as shown.

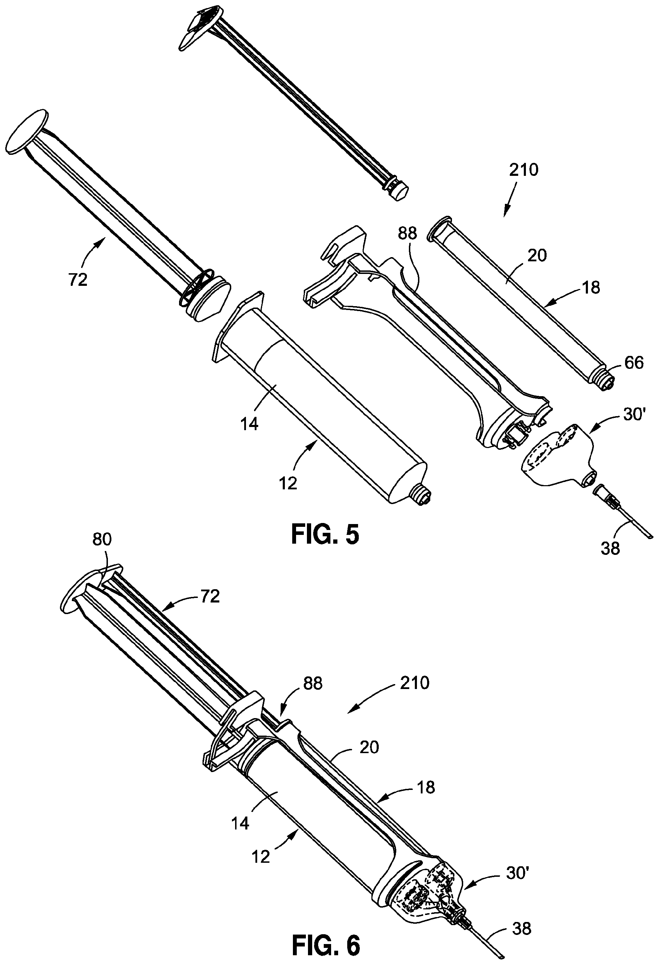

Turning now to Figures 5 and 6, another embodiment is shown generally at 210. This device 210 is similar to device 110, with a major difference being that instead of open sided housing 60 structured for proximal end loading of cartridges 12, 18, device 210 includes a frame housing 88 which allows for side loading, or lateral loading, of cartridges 12, 18. Other features of this embodiment may be identical or similar to corresponding features of device 110 or 10.

For example, for each surgical procedure, tip 30' is snapped onto the housing 88. Cartridge 18 containing additive 20 is placed into the housing 88 by either snapping the cartridge 18 straight in from the side, or by slipping the luer end 66 into side opening of housing 88, then snapping the proximal end of cartridge 18 into the proximal end of housing 88. The cartridge 18 may then be pressed or rotated until it tightens into the tip 30'. The fat cartridge 12 containing adipose material 14 can then be snapped into the housing 88 in a similar manner. The fat cartridge 12 seats into the tip 30' and uses the interference between the existing luer center protrusions of the fat cartridge and the tip 30' for a seal. Manual pressing of the proximal end 80 of plunger assembly 72 causes extrusion of cartridge contents 14, 20 through needle or cannula 38.

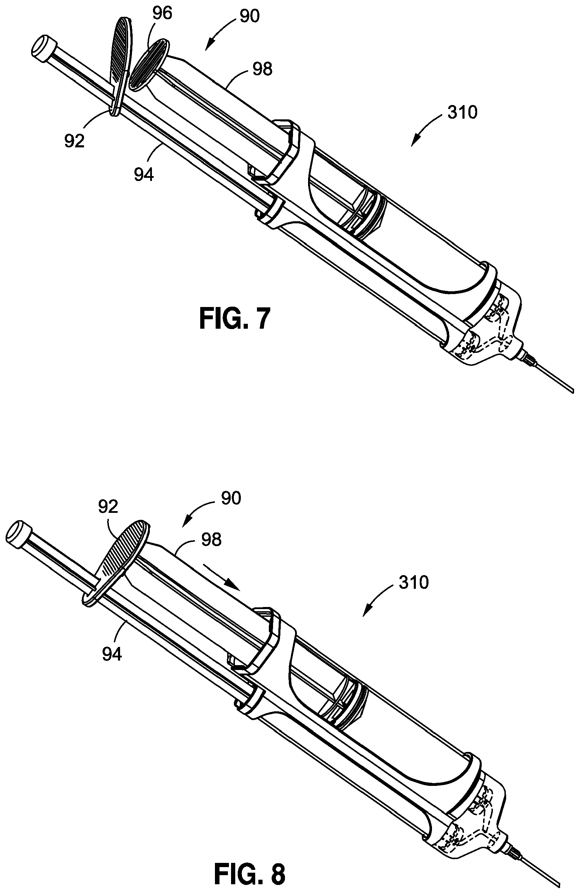

Turning now to Figures 7 and 8, another device in accordance with the invention is shown generally at 310. Device 310 includes a plunger rod assembly 90 having structure for allowing variations in the quantity of harvested adipose material 14. For example, plunger rod assembly 90 may include a fat cartridge plunger rod 98, an additive plunger rod 94, and a variable position plunger rod/thumb piece 92 designed to lock the plunger rods 98, 94 together in variable positions. In the shown embodiment, the thumb piece 92 is pivotally coupled to additive plunger rod 94, and engageable with a corresponding fat cartridge plunger proximal portion 96 of fat cartridge plunger rod 98. The fat cartridge plunger rod 98 may be moved to a desired position and the thumb piece 92 may be then engaged to (e.g. snapped onto) the fat cartridge plunger proximal portion 96, thus fixing the fat cartridge plunger rod 98 and additive cartridge plunger rod 94 in place with respect to each other. This arrangement allows a desired volume injection ratio of fat to additive as the plunger assembly 90 to be maintained while thumb piece 92 is depressed and plunger rods 94, 98 move in unison through cartridges 12, 28. (Fig. 8).

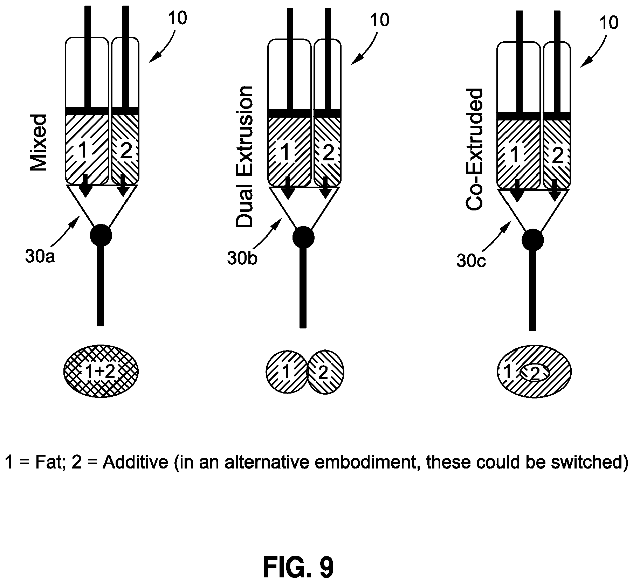

Turning now to Figure 9, it is contemplated that different tips, for example, tips 30a, 30b, and 30c, may be provided on the device 10 (or 110, 210 or 310) of the invention for providing different extrusion/mixing results. Each of these tips 30a, 30b and 30c may serve unique benefits, such as improved vascularization of the fat, decreased extrusion force (for usability), as may be beneficial for different applications on the body.

For example, tip 30a may be provided which includes suitable structure effective to mix or combine the fat material 1 with the additive 2 as these materials 1, 2 are passed through tip 30a immediately prior to extrusion from needle or cannula 38. Alternatively, tip 30b may be provided which is structured to extrude fat material 1 and additive 2 without substantial or significant mixing. Alternatively still, tip 30c may be provided which is structured to provide a co-extrusion of fat material 1 and additive 2, as shown. Although not shown, it can be appreciated that tip 30c may be configured to provide a coextrusion with fat 1 surrounded by additive 2, rather than as shown (additive 2 surrounded by fat 1).

Other tips and structure useful as components of the present devices 10, 110, 210, 310 are disclosed in U.S. Patent Application Serial No. 12/909,216, filed on October 21, 2010, and entitled DUAL CARTRIDGE MIXER SYRINGE, the entire disclosure of which is incorporated herein by this specific reference.

In another aspect of the invention, the device 10, 110, 210, 310 may include a mechanism for discouraging or preventing tampering or misuse of product.

For example, as mentioned hereinabove, the additive cartridge may be permanently fixed in the housing to prevent removal without destruction of the device.

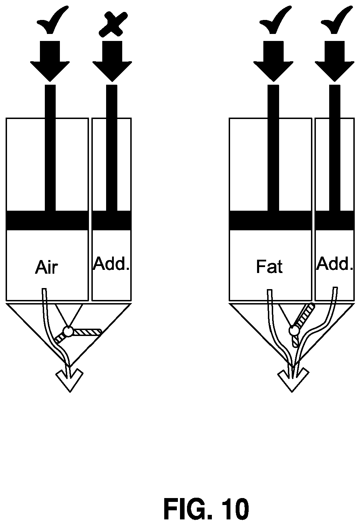

Alternatively or additionally, as illustrated in Figure 10, a feature may be provided which reduces, eliminates or prevents a user from extruding or removing the additive from the device, without also extruding fat material therewith.

For example, the tip 30 or 30' may be provided with a valve 104. Valve 104 includes geometry that allows opening of the additive injection channel only when engineered pressures are induced. Typically air, a highly compressive medium is unable to create a sufficient spike in pressure to rotate the valve and allow dispensing of additive. Alternatively, a medium like fat, a non-compressive medium, is able to create the pressure spike and rotate the valve for additive injection. In other embodiments, the valve could involve flaps, diaphragms, pinches, or other means to achieve the same function. Valve 104 is structured to remain sealed or closed in the event that only air pressure is applied to the fat cartridge valve seat. Thus, the valve 104 would prevent dispensing of additive alone.

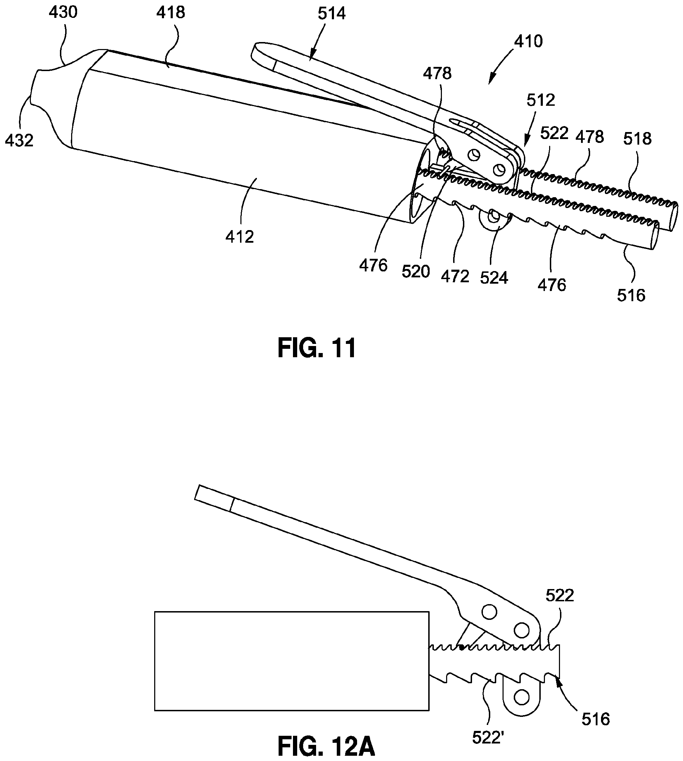

Turning now to Figure 11, another embodiment of the device is shown generally at 410, the device having substantially the same features as devices 10, 110, and 210, but including a ratchet mechanism for operating the plungers.

More specifically, device 410 generally comprises a fat cartridge 412, an additive cartridge 418 and a housing 488 configured to receive, for example, in a side-by-side manner, the fat cartridge 412 and additive cartridge 418. The device further comprises a mixing tip 430 on the housing, the mixing tip including a distal end 432 for receiving a cannula or needle (not shown). A plunger assembly 472 is also provided including a fat cartridge plunger 476 and an additive cartridge plunger 478, configured to be slidably received in the fat cartridge 412 and the additive cartridge 418, respectively. In addition, the device 410 includes a ratchet mechanism 512, including a lever 514 and at least one toothed element connected to, or incorporated as a part of, the plunger assembly 472. In the shown embodiment, the ratchet mechanism 512 comprises two toothed elements 516, 518, one for each plunger 476, 478, which may be proximal extensions of plungers 476, 478. The ratchet mechanism 512 is structured or configured to force the fat cartridge plunger 412 and the additive cartridge plunger 418 in a distal direction upon manual pressure applied to the lever 514, causing pawl 520 to advance the plungers by engaging teeth 522.

The fat cartridge plunger 412 and additive cartridge plunger 418 may be fixed together, for example, by means of the ratchet mechanism 512. In the device 410 shown in Figures 11, the fat cartridge plunger 476 and the additive cartridge plunger 478 are fixed with respect to each other during operation of the device 410, for example, by means of a suitable linkage 524. Thus, upon depression of lever 514, both plungers advance simultaneously. As can be appreciated, the internal diameter of the cartridges can be sized to provide a suitable, consistent mix ratio between fat and additive.

In some embodiments, not shown, the ratchet mechanism comprises a single toothed element (rather than two toothed elements shown), the single toothed element being coupled to both the fat cartridge plunger and the additive cartridge plunger in the cartridges. In this embodiment, this structure is configured to prevent extrusion of additive from the additive cartridge without extrusion of fat from the fat cartridge, and vice versa, as depression of the lever and movement of the single toothed element in a distal direction drives both plungers at the same rate.

In yet other embodiments, the ratchet mechanism comprises dual levers (not shown) which are independently operable to allow dispensing of the contents of the cartridges independently, for example, in an alternating fashion. Thus, a physician can select an amount of fat and an amount of additive to be dispensed into mixing tip, by depressing the levers independently, for example, moving the pawl a desired number of teeth to dispense the contents in a desired fat/additive ratio.

Still referring to Figure 11, the ratchet mechanism 512 includes a mechanical linkage 524. In some embodiments, the toothed element or elements can be rotatable about the longitudinal axis thereof, such that the pawl can engage example, different, for example, larger or differently spaced, teeth on the other side of the element. This will allow for example, a physician to select a desired aliquot of fat and/or additive to be dispensed with each lever stroke. The ratchet mechanism may further include springs or other biasing elements, not shown, which return the plungers to their resting positions.

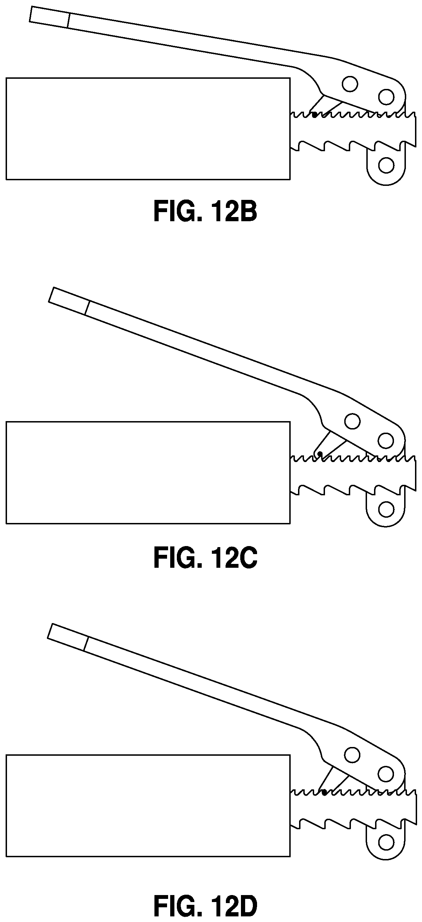

Figures 12A-12D depicts the operation of device 410. Initial position is depicted in 12A, lever depressing and plunger movement in 12B, lever returns to its resting position in 12C, system is ready for a new stroke in 12D. More specifically, in 12A, the device is shown in a "ready" position. The two teeth of the toothed element is behind the syringe body. In 12B, the lever is depressed, and the pawl or pin pushes the plungers forward/distally. Now the plunger has moved "one tooth" forward In 12C, the lever returns to its resting position, due to spring (not depicted). The plungers do not move and the pin starts to go back to its rest position (due to a spring, not depicted) . In 12D, the pin is back in its resting position, and the system is ready for a new stroke.

For a different injection amount, another size of ratchet teeth could be used, either by rotating the plungers so that the pin engages a second, different set of teeth 522', for example, on opposing side of element 516, or by having another lever already engaging those other teeth. The second lever solution provides an "always on" benefit, whereas the rotating plungers solution allow more than two sets of teeth to be used.

Other types of ratcheting systems could also be used, and are considered to be within the scope of the present invention.

The benefits of the ratchet system include providing a mechanical advantage created by the lever, thus reducing the required operator's force to dispense fat/additive, increased precision of the injected amount, different sizes of discrete injection steps, allowing for a more flexible injection experience.

While this invention has been described with respect to various specific examples and embodiments, it is to be understood that the invention is not limited thereto and that it can be variously practiced within the scope of the invention.

## Claims

1. A device (410) for introducing into a target region of a patient, a combination of adipose tissue and an additive, the device comprising:
a fat cartridge (412);
an additive cartridge (418);
a housing (488) configured to receive, in a side-by-side manner, the fat cartridge and additive cartridge;
a mixing tip (430) on the housing, the mixing tip including a distal end (432) for receiving a cannula or needle;
a plunger assembly (472) including a fat cartridge plunger (476) and an additive cartridge plunger (478), configured to be slidably received in the fat cartridge and the additive cartridge, respectively; and **characterized in that** it further comprises
a ratchet mechanism (512), including a lever (514) and at least one toothed element (516, 518) connected to the plunger assembly, the ratchet mechanism configured to force the fat cartridge plunger and the additive cartridge plunger in a distal direction upon manual pressure applied to the lever, thereby causing a pawl (520) or pin to advance the plungers by engaging first teeth (522) on one side of the toothed element, wherein the toothed element is rotatable about the longitudinal axis thereof, such that the pawl or pin is able to engage differently spaced, second teeth on the other side of the toothed element.

2. The device of claim 1 wherein the fat cartridge plunger and the additive cartridge plunger are fixed with respect to each other.

3. The device of claim 2 wherein the fat cartridge plunger and the additive cartridge plunger are fixed together by means of a linkage (524).

4. The device of claim 1, 2 or 3 wherein the housing is configured to receive the fat cartridge and the additive cartridge in a longitudinally slidable manner.

5. The device of claim 1, 2, 3 or 4 wherein the housing is configured to receive the fat cartridge and the additive cartridge in a lateral manner.

6. The device of any preceding claim wherein the ratchet mechanism comprises a single toothed element connected to both the fat cartridge plunger and the additive cartridge plunger to thereby prevent extrusion of additive from the additive cartridge without extrusion of fat from the fat cartridge.

7. The device of claim 1 wherein the additive cartridge contains an additive,
wherein the additive comprises hyaluronic acid and collagen, or
wherein the additive comprises hyaluronic acid crosslinked with collagen.

## Patentansprüche

1. Vorrichtung (410) zum Einbringen einer Kombination von Fettgewebe und einem Zusatz in einen Zielbereich eines Patienten, wobei die Vorrichtung aufweist:
eine Fettkartusche (412);
eine Zusatzkartusche (418);
ein Gehäuse (488), das so ausgestaltet ist, dass es die Fettkartusche und die Zusatzkartusche in einer Seit-an-Seit-Anordnung aufnimmt;
eine Mischspitze (430) am Gehäuse, wobei die Mischspitze ein distales Ende (432) zum Aufnehmen einer Kanüle oder einer Nadel beinhaltet;
eine Kolbenbaugruppe (472), die einen Fettkartuschenkolben (476) und einen Zusatzkartuschenkolben (478) beinhaltet, die so ausgestaltet sind, dass sie in der Fettkartusche beziehungsweise in der Zusatzkartusche verschiebbar aufgenommen werden;
und **dadurch gekennzeichnet ist, dass** sie aufweist:
einen Ratschenmechanismus (512), der einen Hebel (514) und mindestens ein gezahntes Element (516, 518) beinhaltet, das mit der baugruppe verbunden ist, wobei der Ratschenmechanismus so ausgestaltet ist, dass er den Fettkartuschenkolben und den Zusatzkartuschenkolben in eine distale Richtung treibt, wenn ein manueller Druck auf den Hebel aufgebracht wird, und dadurch bewirkt, dass eine Raste (520) oder ein Stift die Kolben durch ein In-Eingriff-Bringen eines ersten Zahnes (522) an einer Seite des gezahnten Elements weiter bewegt, wobei das gezahnte Element um seine Längsachse drehbar ist, sodass die Raste oder der Stift in der Lage ist, mit einem unterschiedlich beabstandeten, zweiten Zahn an der anderen Seite des gezahnten Elements in Eingriff zu treten.

2. Vorrichtung nach Anspruch 1, wobei der Fettkartuschenkolben und der Zusatzkartuschenkolben in Bezug zueinander befestigt sind.

3. Vorrichtung nach Anspruch 2, wobei der Fettkartuschenkolben und der Zusatzkartuschenkolben durch eine Verbindung (524) miteinander befestigt sind.

4. Vorrichtung nach Anspruch 1, 2 oder 3, wobei das Gehäuse so ausgestaltet ist, dass es die erste Fettkartusche und die Zusatzkartusche in einer in einer Längsrichtung verschiebbaren Weise aufnimmt.

5. Vorrichtung nach Anspruch 1, 2, 3 oder 4, wobei das Gehäuse so ausgestaltet ist, dass es die Fettkartusche und die Zusatzkartusche seitlich aufnimmt.

6. Vorrichtung nach einem der vorangegangenen Ansprüche, wobei der Ratschenmechanismus ein einzelnes gezahntes Element aufweist, das sowohl mit dem Fettkartuschenkolben, als auch mit dem Zusatzkartuschenkolben verbunden ist, um dadurch einem Ausstoßen des Zusatzes aus der Zusatzkartusche ohne ein Ausstoßen von Fett aus der Fettkartusche vorzubeugen.

7. Vorrichtung nach Anspruch 1, wobei die Zusatzkartusche einen Zusatz enthält,
wobei der Zusatz Hyaluronsäure und Kollagen enthält, oder
wobei der Zusatz Hyaluronsäure, die mit Kollagen vernetzt ist, enthält.

## Revendications

1. Dispositif (410) pour l'introduction dans une région cible d'un patient, d'une combinaison de tissu adipeux et d'un additif, le dispositif comprenant :
une cartouche de graisse (412) ;
une cartouche d'additif (418) ;
un logement (488) configuré pour recevoir, de manière juxtaposée, la cartouche de graisse et la cartouche d'additif;
une pointe de mélange (430) sur le logement, la pointe de mélange comportant une extrémité distale (432) pour recevoir une canule ou une aiguille ;
un assemblage de piston (472) comportant un piston de cartouche de graisse (476) et un piston de cartouche d'additif (478), configurés pour être reçus en coulissement dans la cartouche de graisse et la cartouche d'additif, respectivement; et **caractérisé en ce que** il comprend en outre
un mécanisme à cliquet (512), comportant un levier (514) et au moins un élément denté (516, 518) connecté à l'assemblage de piston, le mécanisme à cliquet étant configuré pour pousser le pison de cartouche de graisse et le piston de cartouche d'additif dans une direction distale lorsque une pression manuelle est appliquée sur le levier, causant ainsi un cliquet (520) ou une broche à faire avancer les pistons par engagement d'une première dent (522) sur un côté de l'élément denté, dans lequel l'élément denté est rotatif autour de l'axe longitudinal de celui-ci, de telle sorte que le cliquet ou la broche est capable d'engager une deuxième dent espacée différemment sur l'autre côté de l'élément denté.

2. Le dispositif de la revendication 1 dans lequel le pison de cartouche de graisse et le piston de cartouche d'additif sont fixés l'un par rapport à l'autre.

3. Le dispositif de la revendication 2 dans lequel dans lequel le pison de cartouche de graisse et le piston de cartouche d'additif sont fixés ensemble grâce à un moyen de liaison (524).

4. Le dispositif de la revendication 1, 2 ou 3 dans lequel le logement est configuré pour recevoir la cartouche de graisse et la cartouche d'additif d'une manière coulissante longitudinale.

5. Le dispositif de la revendication 1, 2, 3 ou 4 dans lequel le logement est configuré pour recevoir la cartouche de graisse et la cartouche d'additif d'une manière latérale.

6. Le dispositif de l'une quelconque des revendications précédentes dans lequel le mécanisme à cliquet comprend un seul élément denté connecté à la fois au piston de cartouche de graisse et au piston de cartouche d'additif afin d'empêcher l'extrusion d'additif de la cartouche d'additif sans l'extrusion de graisse de la cartouche de graisse.

7. Le dispositif de la revendication 1 dans lequel la cartouche d'additif contient un additif,
dans lequel l'additif comprend de l'acide hyaluronique et du collagène, ou
dans lequel l'additif comprend de l'acide hyaluronique réticulé avec du collagène.
